# DEMANDE DE BREVET EUROPEEN

(11) **EP 1 832 274 A1**
(43) Date de publication de la demande: **12.09.2007**
(21) Numéro de dépôt: 07100911.2
(22) Date de dépôt: 22.01.2007
(51) Int. Cl.: A61K 8/34, A61K 8/86, A61K 8/31, A61K 8/33, A61K 8/37, A61Q 1/14

(54) **Composition gélifiée démaquillante**

(30) Priorité: 06.03.2006 FR 0650774
(71) Demandeur: L'Oréal, 75008 Paris (FR)
(72) Inventeur: Fonolla Moreno , Angeles, 75013 Paris (FR); Jaeger- Paindestre, Pascale, 94260 Fresnes (FR)
(74) Mandataire: Rasson, Catherine

(57) **Abrégé**

L'invention a pour objet une composition cosmétique contenant au moins 40 % en poids d'une ou plusieurs huiles par rapport au poids total de la composition, au moins un ester d'acide gras et de glucose oxyéthyléné, au moins un ester d'acide gras et de polyol oxyéthyléné, au moins un polyol, et de l'eau, la quantité d'eau étant inférieure ou égale à 15 % en poids par rapport au poids total de la composition, et la quantité d'eau et la quantité de polyol étant chacune égale ou supérieure à la quantité d'ester d'acide gras et de glucose oxyéthyléné.

La composition obtenue est transparente et gélifiée. Elle peut être utilisée notamment pour le démaquillage de la peau et/ou de la zone oculaire et/ou des lèvres.

## Description

L'invention a pour objet une composition cosmétique gélifiée contenant des huiles, un émulsionnant dérivé de sucre, un ester de polyol, un polyol et de l'eau, et son utilisation dans le domaine cosmétique et notamment pour le démaquillage de la peau et/ou des yeux.

Les technologies du maquillage actuelles sont de plus en plus innovantes et performantes, et les utilisatrices de produits de maquillage sont de plus en plus nombreuses à utiliser des produits longue tenue tels que des fonds de teint non transferts, des rouges à lèvres de longue tenue, des mascaras waterproof ou des mascaras double geste (application d'une base puis du mascara).

Toutefois, ce type de produits est plus difficile à enlever que les produits classiques de maquillage, et il existe, de ce fait, un besoin de produits démaquillants qui soient à la fois très performants, pratiques à utiliser et qui assurent un respect de la peau tout en ayant de bonnes qualités cosmétiques (bon confort d'utilisation, douceur).

Parmi les produits démaquillants couramment proposés, on peut trouver :
- les technologies classiques telles que les laits démaquillants dont l'efficacité sur le maquillage de longue tenue est limitée et souvent insuffisante.
- Les biphases comprenant une phase aqueuse et une phase huileuse séparées, qui sont émulsionnées l'une dans l'autre seulement au moment de l'utilisation et qui déphasent de nouveau au repos, qui ont une bonne performance mais qui sont liquides et donc coulants, ce qui n'est pas toujours pratique, et qui ne sont pas toujours appréciées pour le démaquillage des yeux du fait que la phase huileuse peut parfois laisser un léger voile huileux sur les yeux.
- Les solutions micellaires à base d'eau et de tensioactifs qui sont très confortables mais peu efficaces vis-à-vis des mascaras waterproof.
- Les huiles démaquillantes qui sont très présentes sur le marché japonais et qui ont une très bonne efficacité sur les produits non transferts mais qui sont peu efficaces sur les produits plus classiques. Par ailleurs, ces huiles étant liquides, leur utilisation est peu pratique puisque le produit risque de couler sur les mains.

Il subsiste donc le besoin de disposer d'un produit de démaquillage, à la fois performant sur tous types de maquillages (classiques et longue tenue), pratique à utiliser et confortable.

Or, la demanderesse a découvert de manière surprenante que l'association de tensioactifs particuliers avec des huiles démaquillantes dans une composition contenant également des pourcentages pondéraux spécifiques d'eau et de polyol par rapport au pourcentage de ces tensioactifs, permettait d'obtenir un gel huileux facile à rincer, l'association de tensioactifs permettant d'une part de gélifier la formule et d'autre part de faciliter le rinçage.

La présente invention a donc pour objet une composition cosmétique contenant au moins 40 % en poids d'une ou plusieurs huiles par rapport au poids total de la composition, au moins un ester d'acide gras et de glucose oxyéthyléné, au moins un ester d'acide gras et de polyol oxyéthyléné, au moins un polyol, et de l'eau, la quantité d'eau étant inférieure ou égale à 15 % en poids par rapport au poids total de la composition, et la quantité d'eau et la quantité de polyol étant chacune égale
ou supérieure à la quantité d'ester d'acide gras et de glucose oxyéthyléné.

La composition selon l'invention présente l'avantage d'être à la fois transparente
ou translucide, et gélifiée, donc facile à utiliser, tant en étant confortable et en permettant un bon démaquillage de tout type de maquillage.. Elle présente en outre une très bonne stabilité.

La transparence de la composition s'apprécie visuellement. On peut voir par exemple que la composition appliquée sur la peau, par exemple en une épaisseur de 5 mm, est transparente, c'est-à-dire laisse voir la peau.

On entend par « gélifiée » le fait que la composition ne coule pas, c'est-à-dire qu'elle ait une certaine viscosité. Sa viscosité peut aller par exemple de 5 à 190 poises (0,5 à 19 Pa.s), de préférence de 5 à 150 poises (0,5 à 15 Pa.s) et mieux de 10 à 120 poises (1 à 12 Pa.s), viscosité mesurée au viscosimètre RHEOMAT 180 à un taux de cisaillement de 200 s⁻¹ et à 25 °C.

Par ailleurs, on entend par « quantité d'eau et la quantité de polyol chacune égale
ou supérieure à la quantité d'ester d'acide gras et de glucose oxyéthyléné » le fait que, indépendamment l'une de l'autre, la quantité d'eau d'une part et la quantité de polyol d'autre part, sont égale ou supérieure à la quantité d'ester d'acide gras et de glucose oxyéthyléné. La quantité d'eau de même que la quantité de polyols doivent être égales ou supérieures à la quantité d'ester d'acide gras et de glucose oxyéthyléné.

La composition selon l'invention est destinée à une application topique et contient donc un milieu physiologiquement acceptable. Par milieu physiologiquement acceptable, on entend un milieu compatible avec la peau, les muqueuses (y compris l'intérieur des paupières, les lèvres), les ongles, la zone oculaire et/ou les fibres kératiniques (cheveux et cils).

La composition selon l'invention contient au moins un ester d'acide gras et de glucose oxyéthyléné. Il s'agit de préférence d'un stéarate de glucose oxyéthyléné, et en particulier du sesquistearate de méthyl glucose à 20 moles d'oxyde d'éthylène, (nom INCI : PEG-20 methyl glucose sesquistearate) comme le produit commercialisé sous la dénomination Glucamate SSE-20 par la société Amerchol et celui commercialisé sous la dénomination Grillocose PSE-20 par la société Goldschmidt.

La quantité d'ester(s) d'acide gras et de glucose oxyéthyléné peut aller par exemple de 0,2 à 15 % en poids, de préférence de 0,5 à 15 % en poids, mieux de 0,5 à 10 % en poids, encore mieux de 1 à 10 % en poids et encore mieux de 2 à 5 % en poids par rapport au poids total de la composition.

La composition contient en outre, un ou plusieurs autres tensioactifs non ioniques choisis parmi les esters d'acides gras et de polyol oxyéthylénés, et de manière préférée, choisis parmi les esters d'acide gras et de glycéryle oxyéthyléné. Comme esters d'acide gras et de glycéryle oxyéthylénés, on peut citer par exemple le triisostéarate de PEG-20 glycéryle (nom INCI : PEG-20 glyceryl triisostearate) comme celui commercialisé par la société Nihon Emulsion sous les noms EMALEX GWIS-305 et EMALEX GWIS-320EX ; et le cocoate de PEG-7 glycéryle (nom INCI : PEG-7 Glyceryl cocoate) comme celui commercialisé par la société Cognis sous le nom CETIOL HE, et leurs mélanges. Selon un mode préféré de réalisation de l'invention, la composition contient du triisostéarate de PEG-20 glycéryle (nom INCI : PEG-20 glyceryl triisostéarate).

La quantité d'ester(s) d'acide gras et de polyol oxyéthyléné peut aller par exemple de 0,5 à 15 % en poids, de préférence de 0,5 à 10 % en poids, mieux de 1 à 10 % en poids, encore mieux de 1 à 5 % en poids et encore mieux de 2 à 4 % en poids par rapport au poids total de la composition.

La composition selon l'invention peut éventuellement contenir un ou plusieurs autres tensioactifs, en particulier des tensioactifs non ioniques, mais seulement dans la mesure où la présence de ces tensioactifs n'affecte pas la transparence et le confort (innocuité) de la composition.

La composition selon l'invention contient une quantité d'eau inférieure ou égale à 15 % en poids par rapport au poids total de la composition. La quantité peut aller par exemple de 0,5 à 15 % en poids, de préférence de 0,6 à 15 % en poids, mieux de 0,6 à 13 % en poids, encore mieux de 1 à 10 % en poids et encore mieux de 3 à 6 % en poids par rapport au poids total de la composition.

L'eau utilisée dans la composition de l'invention peut être de l'eau pure déminéralisée mais aussi de l'eau minérale et/ou de l'eau thermale et/ou de l'eau de mer, c'est-à-dire que l'eau de la composition peut être en partie ou totalement constituée par une eau choisie parmi les eaux minérales, les eaux thermales, les eaux de mer et leurs mélanges. En général, une eau minérale est propre à la consommation, ce qui n'est pas toujours le cas d'une eau thermale. Chacune de ces eaux contient, entre autre, des minéraux solubilisés et/ou des oligo-éléments. Ces eaux sont connues pour être employées à des fins de traitement spécifique selon les oligo-éléments et les minéraux particuliers qu'elles contiennent, tel que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou contenant des oligo-éléments préparées à partir d'eau purifiée (déminéralisée ou distillée).

Une eau thermale ou minérale naturelle utilisée selon l'invention peut, par exemple, être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

La composition de l'invention comprend un ou plusieurs polyols. Parmi les polyols utilisables dans la composition selon l'invention, on peut citer notamment la glycérine, le propylène glycol, le butylène glycol, l'hexylène glycol, les polyéthylène glycols tels que le PEG-8, le dipropylène glycol et leurs mélanges. Selon un mode préféré de réalisation de l'invention, le polyol est la glycérine qui donne une meilleure transparence de la composition que les autres polyols. On peut ajouter à la glycérine, d'autres polyols dans la mesure où les qualités de la composition et notamment la transparence sont maintenues.

La quantité de polyol(s) peut aller par exemple de 0,5 à 15 % en poids, de préférence de 0,5 à 10 % en poids, mieux de 1 à 10 % en poids, encore mieux de 2 à 10 % en poids et encore mieux de 2 à 8 % en poids par rapport au poids total de la composition.

La composition selon l'invention contient au moins une huile, notamment une huile cosmétique.

On entend par "huile" un corps gras liquide à la température ambiante (25°C).

La quantité d'huiles est d'au moins 40 % en poids par rapport au poids total de la composition, de préférence d'au moins 50 % en poids, mieux d'au moins 60 % en poids et encore mieux d'au moins 70 % en poids d'huiles par rapport au poids total de la composition. La quantité d'huiles peut aller notamment de 40 à 98 % en poids, de préférence de 50 à 98 %, mieux de 60 à 95 % en poids et encore mieux de 70 à 90 % en poids par rapport au poids total de la composition.

Comme huiles plus particulièrement utilisables dans la composition de l'invention, on peut citer par exemple :
- les huiles hydrocarbonées d'origine animale, telles que le perhydrosqualène (ou squalane) ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters d'acides gras et les éthers d'alcools gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, la vaseline, les polydécènes, l'isohexadecane, l'isododecane, l'isoparaffine hydrogéné tel que l'huile de Parléam® commercialisée par la société NOF Corporation (nom INCI ; Hydrogenated Polyisobutene) ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- les huiles fluorées telles que celles partiellement hydrocarbonées et/ou siliconées comme celles décrites dans le document JP-A-2-295912 ;
- les benzoates d'alcools gras en C₁₂-C₁₅ tels que ceux commercialisés sous la dénomination Finsolv TN par la société FINETEX ;
- le dicaprylyl carbonate commercialisé sous la dénomination CETIOL CC par la société COGNIS ;
- le dicaprylyl éther commercialisé sous la dénomination CETIOL OE par la société COGNIS,
- et leurs mélanges.

De préférence, la composition contient au moins une huile polaire qui peut être choisie notamment parmi les huiles d'origine végétale, les esters et éthers d'acide gras, et leurs mélanges.

Quand la composition de l'invention est utilisée comme composition démaquillante, elle contient de préférence au moins une huile démaquillante, à laquelle on peut ajouter une ou plusieurs autres huiles démaquillantes ou non.

Les huiles démaquillantes peuvent être choisies notamment parmi les hydrocarbures ramifiés décrits ci-dessus, les esters d'acide gras décrits ci-dessus, et leurs mélanges.

Les esters d'acide gras sont de préférence choisis parmi les esters obtenus à partir d'un alcool à chaîne linéaire ou ramifiée, ayant de 1 à 17 atomes de carbone et d'un acide gras à chaîne linéaire ou ramifiée, ayant de 3 à 18 et de préférence de 12 à 17 atomes de carbone.

Comme esters d'acide gras utilisables comme huiles démaquillantes, on peut citer plus particulièrement le palmitate d'éthyl hexyle, le stéarate d'éthyl hexyle, le myristate d'isopropyle, le palmitate d'isopropyle, le palmitate d'isobutyle, le caprate/caprylate de pentaérythritol, l'isononanoate de cétéaryle, l'isononanoate d'isodécyle, l'isononanoate d'isononyle, l'isononanoate d'isotridécyle, le caprate/caprylate d'éthyl-2 hexyle.

Selon un mode particulier de réalisation de l'invention, la composition peut contenir en outre au moins une huile choisie parmi les hydrocarbures ramifiés, d'origine minérale ou synthétique, tels que, notamment, l'isoparaffine, l'isohexadecane, l'isododecane, le polyisobutène hydrogéné tel que l'huile de Parléam®.

Il peut être avantageux aussi que la composition selon l'invention contienne un ester à chaîne courte tels que le dicaprylyl carbonate ou un éther à chaîne courte tel que la dicaprylyl éther, pour améliorer l'efficacité de démaquillage de la composition.

Les compositions cosmétiques de l'invention peuvent, en outre, contenir des adjuvants habituels dans le domaine cosmétique, tels que les antioxydants, les parfums, les peptisants de parfums, les matières colorantes, les actifs hydrophiles
ou lipophiles. La nature des adjuvants et leurs quantités doivent être telles qu'elles ne modifient pas les propriétés de la composition selon l'invention. Les quantités de ces adjuvants sont celles classiquement utilisées dans le domaine cosmétique et par exemple de 0,001 à 10 % du poids total de la composition.

Comme actifs utilisables dans la composition de l'invention, on peut citer par exemple les agents apaisants comme l'allantoïne et le bisabolol ; les eaux florales telles que l'eau de tilleul ou l'eau de bleuet ; l'acide glycyrrhétinique et ses sels ; les antibactériens comme l'octopirox, le triclosan et le triclocarban ; les huiles essentielles ; les vitamines telles que par exemple le rétinol (vitamine A), l'acide ascorbique (vitamine C), le tocophérol (vitamine E), la niacinamide (vitamine PP
ou B3), le panthénol (vitamine B5) et leurs dérivés tels que par exemple les esters de ces vitamines (palmitate, acétate, propionate), l'ascorbyl phosphate de magnésium, la vitamine C glycosylée ou acide glucopyranosyl ascorbique (Ascorbyl glucoside) ; les co-enzymes tels que le co-enzyme Q10 ou ubiquinone et le co-enzyme R ou biotine ; les hydrolysats de protéine ; les extraits végétaux et notamment les extraits de plancton ; et leurs mélanges.

Bien entendu, l'homme de l'art veillera à choisir le ou les éventuels additifs à ajouter à la composition selon l'invention de manière telle que les propriétés avantageuses attachées intrinsèquement à la composition conforme à l'invention ne soient pas, ou substantiellement pas, altérées par l'addition envisagée.

Les compositions de l'invention peuvent être utilisées dans toute application cosmétique. Elles sont particulièrement adaptées au démaquillage de la peau et/ou de la zone oculaire et/ou des lèvres.

Aussi, l'invention a encore pour objet l'utilisation cosmétique de la composition telle que définie ci-dessus pour le démaquillage de la peau et/ou de la zone oculaire et/ou des lèvres.

Le mode d'utilisation lors du démaquillage consiste à appliquer la composition sur la peau et à rincer ensuite la peau.

Les exemples suivants sont donnés à titre d'illustration de l'invention et n'ont pas de caractère limitatif. Toutes les quantités sont données en pourcentage en poids par rapport au poids total de la composition. Les noms des composés sont indiqués selon les cas en noms chimiques ou en noms INCI.

### Exemple 1 selon l'invention : huile démaquillante

| *Phase I* | |
|---|---|
| Eau | 3,5 % |
| Glycérine | 4,5 % |

| | |
|---|---|
| *Phase II* | |
| PEG-20 methyl glucose sesquistearate | 2 % |
| PEG-20 glyceryl triisostearate | 1 % |
| Caprylic/capric triglyceride | qsp 100 % |
| Isohexadecane | 20 % |
| Ethylhexyl stearate | 20 % |

### Mode opératoire : on a chauffé à 45°C la phase Il puis, quand la phase Il a été homogène, on l'a refroidie à 25°C et on l'a ensuite introduite dans la phase I sous agitation à la turbine.

On a obtenu une composition transparente gélifiée, qui ne coulait pas et qui était bien stable. Cette composition s'est avérée très confortable lors de l'utilisation et elle a présenté une très bonne efficacité de démaquillage.

Cette composition peut être utilisée par exemple par application directe sur le visage avec les doigts ou un coton, puis rinçage.

### Exemple 2 selon l'invention : huile démaquillante

| *Phase I* | |
|---|---|
| Eau | 5 % |
| Glycérine | 10 % |
| | |

| *Phase II* | |
|---|---|
| PEG-20 methyl glucose sesquistearate | 3,5 % |
| PEG-20 glyceryl triisostearate | 3 % |
| Dicaprylyl carbonate (Cetiol CC) | 10 % |
| Isododecane | qsp100 % |
| Ethylhexyl palmitate | 20 % |

### Mode opératoire : identique à celui de l'exemple 1.

On a obtenu une composition transparente gélifiée, qui ne coulait pas et qui était bien stable. Cette composition s'est avérée très confortable lors de l'utilisation et elle a présenté une très bonne efficacité de démaquillage.

Son mode d'utilisation est le même que pour l'exemple 1.

### Exemple 3 selon l'invention:

| *Phase I* | |
|---|---|
| Eau | 3 % |
| Glycérine | 5,5 % |
| | |

| *Phase II* | |
|---|---|
| PEG-20 methyl glucose sesquistearate | 2,5 % |
| PEG-20 glyceryl triisostearate | 4 % |
| Caprylic/capric triglycerides | qsp 100 % |
| Dicaprylyl ether | 3 % |
| Isohexadecane | 15 % |
| Ethylhexyl stéarate | 15 % |
| Acétate de tocophérol | 0,1 % |

### Mode opératoire : identique à celui de l'exemple 1.

Son mode d'utilisation est le même que pour l'exemple 1.

La composition obtenue est gélifiée et présente une très bonne stabilité.

### Exemple comparatif 1 :

| *Phase I* | |
|---|---|
| Eau | 0,5 % |
| Glycérine | 5 % |
| | |

| *Phase II* | |
|---|---|
| PEG-20 methyl glucose sesquistearate | 2 % |
| PEG-20 glyceryl triisostearate | 4 % |
| Isononanoate d'isononyle | qsp 100 % |
| Isododecane | 40 % |
| Ethylhexyl stéarate | 30 % |

### Mode opératoire : identique à celui de l'exemple 1.

On a obtenu une composition qui présentait une mauvaise stabilité puisqu'on a observé un déphasage au bout de 48 heures (séparation de l'huile et l'eau avec relargage de l'huile) au lieu d'obtenir un gel transparent stable.

Cet exemple comparatif montre que, lorsque la quantité d'eau est inférieure à celle du tensioactif dérivé de glucose (PEG-20 methyl glucose sequistearate), on n'atteint pas le but de l'invention. Le résultat est le même quand la quantité de glycérine est inférieure à la quantité de ce tensioactif.

### Exemple comparatif 2:

| *Phase I* | |
|---|---|
| Eau | 3 % |
| Glycérine | 5,5 % |
| | |

| *Phase II* | |
|---|---|
| PEG-20 methyl glucose sesquistearate | 2,5 % |
| Caprylic/capric triglycerides | qsp 100 % |
| Dicaprylyl ether | 3 % |
| Isohexadecane | 15 % |
| Ethylhexyl stéarate | 15 % |
| Acétate de tocophérol | 0,1 % |

### Mode opératoire : identique à celui de l'exemple 1.

On a obtenu une composition qui présentait une mauvaise stabilité puisqu'on n'a pas pu incorporer toute l'huile et qu'on a observé un déphasage dès la préparation.

Cet exemple comparatif montre que, lorsque la composition ne contient pas d'ester d'acide gras et de polyol oxyéthyléné, la composition obtenue n'est pas stable et ne peut se faire alors qu'en présence d'ester d'acide gras et de polyol oxyéthyléné (exemple 3 selon l'invention), on peut réaliser la composition et elle est stable.

## Revendications

1. Composition cosmétique contenant au moins 40 % en poids d'une ou plusieurs huiles par rapport au poids total de la composition, au moins un ester d'acide gras et de glucose oxyéthyléné, au moins un ester d'acide gras et de polyol oxyéthyléné, au moins un polyol, et de l'eau, la quantité d'eau étant inférieure ou égale à 15 % en poids par rapport au poids total de la composition, et la quantité d'eau et la quantité de polyol étant chacune égale ou supérieure à la quantité d'ester d'acide gras et de glucose oxyéthyléné.

2. Composition selon la revendication 1, **caractérisée en ce que** l'ester d'acide gras et de glucose oxyéthyléné est le sesquistearate de méthyl glucose à 20 moles d'oxyde d'éthylène.

3. Composition selon la revendication 1 ou 2, **caractérisée en ce que** la quantité d'ester(s) d'acide gras et de glucose oxyéthyléné va de 0,5 à 15 % en poids par rapport au poids total de la composition.

4. Composition selon l'une quelconque des revendications précédentes, **caractérisée en ce que** l'ester d'acide gras et de polyol oxyéthyléné est un ester d'acide gras et de glycéryle oxyéthyléné.

5. Composition selon la revendication précédente, **caractérisée en ce que** l'ester d'acide gras et de glycéryle oxyéthyléné est le triisostéarate de PEG-20 glycéryle.

6. Composition selon l'une quelconque des revendications précédentes, **caractérisée en ce que** la quantité d'ester(s) d'acide gras et de polyol oxyéthyléné va de 0,5 à 15 % en poids par rapport au poids total de la composition.

7. Composition selon l'une quelconque des revendications précédentes, **caractérisée en ce que** le polyol est la glycérine.

8. Composition selon l'une quelconque des revendications précédentes, **caractérisée en ce que** la quantité de polyol(s) va de 0,5 à 15 % en poids par rapport au poids total de la composition.

9. Composition selon l'une quelconque des revendications précédentes, **caractérisée en ce que** la quantité d'huiles va de 40 à 98 % en poids par rapport au poids total de la composition.

10. Composition selon l'une quelconque des revendications précédentes, **caractérisée en ce qu'**elle contient au moins une huile polaire.

11. Composition selon l'une quelconque des revendications précédentes, **caractérisée en ce qu'**elle contient au moins une huile choisie parmi les huiles d'origine végétale, les esters d'acide gras et les éthers d'alcools gras, et leurs mélanges.

12. Utilisation cosmétique de la composition selon l'une quelconque des revendications 1 à 11, pour le démaquillage de la peau et/ou de la zone oculaire et/ou des lèvres.
